(19) Europäisches Patentamt
European Patent Office
Office européen des brevets

(11) Veröffentlichungsnummer: **0 216 185**
**B1**

# (12) EUROPÄISCHE PATENTSCHRIFT

(45) Veröffentlichungstag der Patentschrift: **02.11.88**

(51) Int. Cl.⁴: **C 07 D 303/04**, C 07 D 301/14, A 61 K 7/46

(21) Anmeldenummer: **86111892.5**

(22) Anmeldetag: **28.08.86**

(54) **16-Oxa-bicyclo 13.1.0 -hexadec-7-en, Verfahren zu dessen Herstellung und dessen Verwendung als Riechstoff.**

(30) Priorität: **29.08.85 DE 3530885**

(43) Veröffentlichungstag der Anmeldung: **01.04.87 Patentblatt 87/14**

(45) Bekanntmachung des Hinweises auf die Patenterteilung: **02.11.88 Patentblatt 88/44**

(84) Benannte Vertragsstaaten: **CH DE FR GB LI NL**

(56) Entgegenhaltungen:
**DE-B-1 693 162**
**US-A-3 333 010**
**US-A-4 380 658**

(73) Patentinhaber: **Consortium für elektrochemische Industrie GmbH, Zielstattstrasse 20, D-8000 München 70 (DE)**

(72) Erfinder: **Eberle, Hans- Jürgen, Dr. Dipl.- Chem., Höglwörtherstrasse 351, D-8000 München 70 (DE)**
Erfinder: **Gebauer, Helmut, Dr. Dipl.- Chem., Schaffhauser Strasse 18/7, D-8000 München 71 (DE)**

**EP 0 216 185 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Als Riechstoffe geeignete höhere Epoxycycloalkene sind z. B. aus US-A-3 333 010 und DE-B-1 693 162 bekannt.

Gegenstand der Erfindung ist 16-Oxa-bicyclo[13.1.0]-hexadec-7-en. Die genannte Verbindung kann durch die folgende Strukturformel dargestellt werden:

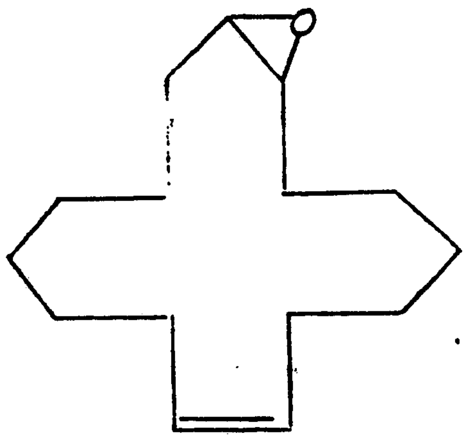

Die erfindungsgemäße Verbindung ist durch partielle Oxidation von Cyclopentadeka-1.8-dien mit Persäuren zugänglich.

Das Verfahren zur Herstellung der erfindungsgemäßen Verbindung ist dadurch gekennzeichnet, daß Cyclopentadeka-1.8-dien mittels organischer Persäure umgesetzt wird.

Beispiele für erfindungsgemäß einzusetzende Persäuren sind Perameisensäure, Peressigsäure, meta-Chlorperbenzoesäure und andere. Die Herstellung bzw. in situ-Herstellung dieser Perverbindungen ist Stand der Technik.

Das Ausgangsprodukt Cyclopentadekadien-1.8 ist eine an sich bekannte Verbindung. Sie ist beispielsweise durch Cometathese von Cycloocten und Cyclohepten an Metathesekatalysatoren wie beispielsweise Rheniumheptoxid zugänglich.

Cyclopentadekadien-1.8 und Persäure werden in etwa äquimolaren Mengen zur Reaktion gebracht. In der Regel beträgt das molare Verhältnis von Dien zu Persäure 1 : 0,7 bis 1,1, insbesondere 1 : 0,8 bis 1.

Zumeist wird, ohne daß dies zwingend erforderlich wäre, in inerten Lösungsmitteln wie Methylenchlorid, Chloroform, Tetrachlorkohlenstoff, Dichlorethan, und anderen gearbeitet.

Um die Epoxidierung der zweiten Olefinbindung zu unterdrücken, wird zweckmäßigerweise das Dien vorgelegt und die Persäure zudosiert.

Die erfindungsgemäße Verbindung findet Verwendung als Duftstoff. Sie bietet ein Geruchsbild, in dem warme und animalische Noten hervortreten und sich Ambra-, Moschus- und Holznoten harmonisch vereinen. Weiterhin verfügt der erfindungsgemäße Duftstoff über sehr gute Haft- und Pixiereigenschaften. Der Duftstoff wird zur Parfümierung von kosmetischen und technischen Produkten eingesetzt. Aufgrund seines hochwertigen Duftcharakters eignet sich der Duftstoff insbesondere zur Verwendung als Fixateur und Basisnote in der Feinparfümerie.

Ferner kann die erfindungsgemäße Verbindung als Ausgangsstoff zur Synthese der ihrerseits wiederum als Duftstoffe verwendbaren Ketone Cyclopentadec-7-en-1-on und Cyclopentadec-8-en-1-on eingesetzt werden. Die Umwandlung des Epoxids zum entsprechenden Keton kann nach an sich bekannten Methoden, beispielsweise durch Behandeln des Epoxids mit Säuren bzw. Lewis-Säuren erfolgen.

Die Erfindung wird nun anhand von Beispielen näher erläutert:

**Beispiel 1**

Herstellung von 16-Oxa-bicyclo[13.1.0]-hexadec-7-en

Zu einer Mischung aus 100 g (0.49 mol) Cyclopentadeca-1.8-dien, 217 g (1,6 mol) Natriumacetat x 3 $H_2O$ und 1000 ml Methylenchlorid wurden unter Rühren innerhalb von 2 Stunden 78 ml 40 gewichtsprozentige (0,47 mol) Peressigsäure zugetropft. Die Reaktionstemperatur betrug 2°C. Danach wurde bei der gleichen Temperatur noch 30 Minuten gerührt und anschließend auf Raumtemperatur erwärmt. Nach weiterem zweistündigem Rühren bei Raumtemperatur wurde das Reaktionsgemisch zunächst mit 5 x 100 ml Wasser, anschließend mit 100 ml 5 gewichtsprozentiger Natriumbicarbonatlösung und schließlich mit 100 ml gesättigter Natriumchloridlösung neutral und peroxidfrei gewaschen. Danach wurden die Phasen getrennt. Die organische Phase wurde über Natriumsulfat getrocknet. Nach Entfernen des Lösungsmittels wurde das Produktgemisch

fraktioniert destilliert. Es wurden 53 g an Zielprodukt erhalten neben 35 g Ausganssubstanz (Cyclopentadeca-1.8-dien) und 11 g Cyclopentadecadiendioxid.

Das Zielprodukt wird durch die folgenden Daten charakterisiert:

Siedepunkt bei 0,013 mbar 100°C

Massenspektrum: m/e (Intensität, %) 222 (I, Molekülion), 204(0.4) 165 (7), 147 (7), 121 (15), 109 (25), 95 (41), 81 (60), 67 (95), 41 (100)

**Beispiel 2**

Verwendung von 16-Oxa-bicyclo[13.1.0]-hexadec-7-en als Duftstoff. Blumige Phantasiekomposition

| | a | b |
|---|---|---|
| Synambran 1 % in DPG (Tetramethylperhydronaphthofuran) | 40 | 40 |
| Dihydromyrcenol | 60 | 60 |
| Orangenöl bras. | 60 | 60 |
| Nerolialdehyd (2,5-Dimethyl-2-vinyl-4-hexenal) | 20 | 20 |
| Lavendelöl Barréme | 60 | 60 |
| Benzylacetat | 60 | 60 |
| Hexylzimtaldehyd alpha | 140 | 140 |
| Jaswalia (4-acetoxy-3-pentyl-2H-tetrahydopyran) | 80 | 80 |
| Methylionon gamma | 60 | 60 |
| Phenylethanol | 110 | 110 |
| Nelkenöl, Sansibar | 15 | 15 |
| Benzylsalicylat | 45 | 45 |
| Lignoxan (14-Methyl-13-oxa-bicyclo-(10,3,0)-pentadecane) | 30 | 30 |
| Galbanumöl 10 % in DPG | 30 | 30 |
| Sandelholzöl | 40 | 40 |
| Dipropylenglykol | 60 | - |
| Cyclopentadekadienoxid | - | 60 |
| | --- | --- |
| | 1000 | 1000 |

Die Zusammensetzung a) ist eine blumige Phantasiekomposition. Durch Zugabe von 60 Gewichtsteilen des erfindungsgemäßen Duftstoffes wird ein Parfümöl mit einer warmen, natürlichen Moschusnote unter Betonung holzig, ambrierter Aspekte erhalten. Das Geruchsbild ist feiner und abgerundet.

**Patentansprüche**

1. 16-Oxa-bicyclo-[13.1.0]-hexadec-7-en.

2. Verfahren zur Herstellung der Verbindung nach Anspruch 1, dadurch gekennzeichnet, daß Cyclo pentadeca-1.8-dien mittels organischer Persäure epoxidiert wird.

3. Verwendung der Verbindung nach Anspruch 1 als Duftstoff.

**Claims**

1. 16-Oxa-bicyclo[13.1.0]-hexadec-7-ene.

2. Process for the preparation of the compound according to claim 1, characterized in that cyclopentadeca-1,8-diene is epoxidized by means of organic peracid.

3. Use of the compound according to Claim 1 as a fragrance.

**Revendications**

1. Oxa-16 bicyclo[13.1.0]-hexadécène-7.

2. Procédé de préparation du composé selon la revendication 1, procédé caractérisé en ce qu'on époxyde le cyclopentadécadiène-1,8 au moyen d'un peracide organique.

3. Application du composé selon la revendication 1 comme parfum.